(19) **Europäisches Patentamt / European Patent Office / Office européen des brevets**

(11) **EP 4 130 735 B1**

(12) **EUROPEAN PATENT SPECIFICATION**

(45) Date of publication and mention of the grant of the patent:
**18.02.2026 Bulletin 2026/08**

(21) Application number: **21776633.6**

(22) Date of filing: **05.03.2021**

(51) International Patent Classification (IPC):
**G01N 33/00** $^{(2006.01)}$

(52) Cooperative Patent Classification (CPC):
**G01N 33/0034; G01N 33/0001**

(86) International application number:
**PCT/JP2021/008633**

(87) International publication number:
**WO 2021/192915 (30.09.2021 Gazette 2021/39)**

(54) **PRIMARY ODOR SELECTION METHOD, METHOD FOR EXPRESSING, PRESENTING, OR SYNTHESIZING ODOR FROM COMBINATION OF PRIMARY ODORS, AND DEVICE FOR SAME**

VERFAHREN ZUR AUSWAHL PRIMÄRER GERÜCHE, VERFAHREN ZUR EXPRESSION, DARSTELLUNG ODER SYNTHESE VON GERÜCHEN AUS EINER KOMBINATION PRIMÄRER GERÜCHE UND VORRICHTUNG DAFÜR

PROCÉDÉ DE SÉLECTION D'ODEUR PRIMAIRE, PROCÉDÉ D'EXPRESSION, DE PRÉSENTATION OU DE SYNTHÉTISE D'UNE ODEUR À PARTIR D'UNE COMBINAISON D'ODEURS PRIMAIRES, ET DISPOSITIF ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2020 JP 2020051050**

(43) Date of publication of application:
**08.02.2023 Bulletin 2023/06**

(73) Proprietor: **National Institute for Materials Science**
**Tsukuba-shi, Ibaraki 305-0047 (JP)**

(72) Inventors:
- **TAMURA, Ryo**
  **Tsukuba-shi, Ibaraki 305-0047 (JP)**
- **XU, Hanxiao**
  **Tsukuba-shi, Ibaraki 305-0047 (JP)**
- **KITAI, Koki**
  **Tsukuba-shi, Ibaraki 305-0047 (JP)**
- **MINAMI, Kosuke**
  **Tsukuba-shi, Ibaraki 305-0047 (JP)**
- **NAKATSU, Makito**
  **Tsukuba-shi, Ibaraki 305-0047 (JP)**
- **YOSHIKAWA, Genki**
  **Tsukuba-shi, Ibaraki 305-0047 (JP)**
- **SHIBA, Kota**
  **Tsukuba-shi, Ibaraki 305-0047 (JP)**
- **TSUDA, Koji**
  **Tsukuba-shi, Ibaraki 305-0047 (JP)**

(74) Representative: **Dehns**
**10 Old Bailey**
**London EC4M 7NG (GB)**

(56) References cited:
| | |
|---|---|
| WO-A1-00/15268 | WO-A1-2016/031080 |
| WO-A1-2018/211642 | WO-A1-2018/221283 |
| WO-A1-2019/111262 | JP-A- 2002 524 205 |
| JP-A- 2002 524 206 | JP-A- 2013 124 953 |
| US-A1- 2016 216 244 | |

- **WEN TENGTENG ET AL: "The Odor Characterizations and Reproductions in Machine Olfactions: A Review", SENSORS, vol. 18, no. 7, 18 July 2018 (2018-07-18), CH, pages 2329, XP093137037, ISSN: 1424-8220, DOI: 10.3390/ s18072329**

• TAKAMICHI NAKAMOTO: "Study of odor sensing and its reproduction", THE JAPANESE JOURNAL OF TASTE AND SMELL RESEARCH, vol. 19, no. 2, 31 July 2012 (2012-07-31), pages 147 - 155, XP009539471, ISSN: 2424-1326, DOI: 10.18965/tasteandsmell.19.2_147

**Description**

Technical Field

The present invention relates to selecting, from among an odor set consisting of a plurality of kinds of odors, a subset of the odor set, which is a subset consisting of a relatively small number of odors and represents any odor in the odor set in an at least approximate manner by using the combination thereof (hereinafter, each individual odor in this subset is referred to as a primary odor). The present invention also relates to representing, presenting, or synthesizing many kinds of odors in an at least approximate manner by using the combination of the primary odors thus selected. The present invention further relates to representing or presenting many kinds of odors in a form perceivable by a perception other than olfaction, for example, in color. The present invention further relates to an apparatus therefor.

Background Art

As is well known, any color can be separated into three primary colors and represented by using weights for the respective primary colors. Furthermore, any color can be synthesized so as to be restored on human vision by mixing the light of the respective primary colors with the intensity of the weight corresponding to the color to be represented. In principle, such synthesis requires to prepare only three kinds of colors instead of preparing the light emission source or the colorant of every color. Drawing from the analogy of separating or synthesizing a color into or from primary colors as described above, it is conceivable that any odor is separated into a relatively small number of odors, namely, "primary odors" corresponding to primary colors, and any odor is synthesized by mixing such primary odors with weight. If such separation and synthesis of odor is achieved, a huge number of applications will be expected.

Note that, as for the primary odor, separating an odor into the primary odors or synthesizing an odor by mixing some primary odors does not mean separation or synthesis in the sense of the component composition of odor. It should be noted that the primary odor means an original odor from which an odor identical or very similar to any target odor in terms of olfaction (human or other animal olfaction, or more generally response of various odor sensors) can be synthesized by mixing themselves. For example, even if an odor X of gas containing acetic acid can be synthesized by mixing a primary odor A and a primary odor B, it does not necessarily mean that acetic acid is contained in any of the primary odors A and B. It merely means that gas obtained by mixing these primary odors at a certain ratio is perceived as an odor identical or similar to the odor X.

Furthermore, when an odor is converted into a form in which the odor can be recognized by a kind of perception other than olfaction by separating the odor into the primary odors, the odor can be represented more in an easy-to-understand manner. Consider an application in a case, for example, where an odor can be represented by color. If odor can be colored, the effect of "odor" can be used in comics and movies. In addition, if perfumes, cosmetics, whiskey, wine, and the like can be represented by colors, it is possible to visually understand the odor of a product and contribute to sales by adopting the colors in a package of the product. Since color has an effect of influencing human mind, applications are limitless if odor can be represented by color. Furthermore, since a method for representing odor itself has not been established so far, odor can be represented more objectively than before by representing odor as color. It is expected that this makes it easier to store, learn, and understand odor.

However, it is generally difficult to represent odor by separation of odor into the primary odors as described above, synthesize and generate odor by mixture, and convert odor into a form that can be recognized by other perception. This is because the primary odors in olfaction corresponding to the three primary colors of light and the four primary tastes (some say that the number of primary tastes is five, six, or more) is not scientifically identified. Studies for trying to define the primary odor have been conducted for a long time, and Henning's odor prism and the like are famous. Henning's odor prism is proposed by Henning, and in the odor prism, six kinds of flowery, fruity, putrid, spicy, resinous, and burnt are regarded as primary odors. However, when the sample as a primary odor is collected, not all odors can be represented by a mixed odor of these primary odors. In case of the light or taste, any other light or taste can be reproduced by preparing a reference sample, but odor cannot. For example, when it is desired to represent an odor by a single color, three primary odors corresponding to three primary colors of light are required to be found, but from the above reason, it is substantially impossible to determine the three primary odors.

By the way, development of an artificial olfactory system using a sensor array is remarkable in recent years. By using such an artificial olfactory system, it is becoming possible to numerically represent odor. Further, studies for visualizing odor using a sensor array have also been conducted by using various methods, and odor is generally represented as a set of a plurality of colors. Among these studies, use of an optoelectronic nose is the mainstream. In this method, materials, such as a plurality of dyes and pigments, whose colors change when the odor component comes into contact with the materials are prepared, and changes in color when an odor to be targeted is blown onto the materials are detected. However, in these strategies, the primary odor has not been investigated, and odor cannot be represented by a single color.

For example, Non Patent Literature 1 discloses "absolute value expression analysis" in which a comparison result with several kinds of reference gasses defined in advance is quantified and expressed on a radar chart or the like, and an example of such quantification and the radar chart expression is disclosed in Non Patent Literature 2. However, in the absolute value expression described in the above literature, it is necessary for the provider or the user of the measurement system to preset the reference odor (reference gas), but whether or not the odor to be measured can be favorably approximated by using the combination of these reference gasses has not been examined. Therefore, the absolute value expression analysis disclosed in the above literature does not give any suggestion about the above-described odor representation by the separation of odor into the primary odors, synthesis and generation by mixture, and conversion into a form that can be recognized by other perception.

Patent Literature 2 discloses the construction of an odorant palette by selecting from the set of all input odors a subset of odors corresponding the most principal odor components.

Summary of Invention

Technical Problem

The problem to be solved by the present invention is to systematically select primary odors from a set of odors. Another problem to be solved by the present invention is to separate, synthesize/generate any odor by using the selected primary odors. Still another problem to be solved by the present invention is to represent or present odor by a single color by using the selected primary odors. Yet another problem to be solved by the present invention is to produce a device capable of converting odor into color in real time.

Solution to Problem

According to one aspect of the present invention, there is provided a method for selecting primary odors as defined in claim 1, comprising, inter alia: detecting each of odors in a set of the odors; and selecting a subset consisting of primary odors from the set of the odors, based on detection results of each of the odors.

The detecting each of the odors includes obtaining a feature vector corresponding to each of the odors, each of the feature vectors comprising a plurality of features obtained from output signals of a sensor for each of the odors, and the selecting the subset consisting of the primary odors includes selecting the primary odors from a set of the feature vectors respectively corresponding to the odors in the set of the odors, based on a subset of the feature vectors selected by endpoint detection in feature space including the feature vectors.

Each row in a feature matrix may be subjected to scale conversion, the feature matrix being constituted by taking the feature vectors obtained corresponding to the respective odors as column vectors thereof.

The subset of the feature vectors is obtained by counting the number of times the respective feature vectors are end points, the end point being a point located at an extreme end in any arbitrary direction in the feature space, and extracting the feature vectors from the set of the feature vectors in descending order of the counted number of times.

The sensor may be a surface stress sensor.

The sensor may be a sensor array having a plurality of sensor elements, and the output signals may include individual output signals from the respective plurality of sensor elements.

According to another aspect of the present invention, there is provided an apparatus for selecting primary odors according to claim 12, comprising: a sensor; a gas supply means configured to alternately supply sample gases and reference gas to the sensor, each of the sample gases having an odor corresponding to each of odors in a set of the odors; and an information processing device configured to input output signals from the sensor, wherein the information processing device is configured to select some odors of the sample gases as primary odors by using the above-described method for selecting primary odors, each of the some odors selected corresponding to each element of the subset of the feature vectors obtained by the information processing device.

According to still another aspect of the present invention, there is provided a method for synthesizing an odor comprising synthesizing, based on a plurality of primary odors selected by using the above-described method for selecting primary odors, a given odor as an odor obtained by mixing the primary odors selected.

According to yet another aspect of the present invention, there is provided an apparatus for synthesizing an odor comprising a means configured to mix the sample gases corresponding to each of the primary odors selected by using the apparatus for selecting primary odors.

According to yet another aspect of the present invention, there is provided a method for representing an odor comprising representing, based on a plurality of primary odors selected by using the above-described method for selecting primary odors, a given odor as a combination of the primary odors selected.

The combination of the primary odors may be represented by a linear combination of feature vectors of the primary odors.

According to yet another aspect of the present invention, there is provided an apparatus for representing an odor comprising: a sensor; a gas supply means configured to alternately supply sample gases and reference gas to the sensor, each of the sample gases having an odor corresponding to each of odors in a set of the odors; an information processing device configured to input output signals from the sensor; and a means configured to store a result of odor representation obtained by the information processing device by using the above-described method for representing odor, or configured to provide the result of odor representation to an outside.

According to yet another aspect of the present invention, there is provided a method for presenting an odor comprising presenting the combination of the primary odors in a form recognizable by perception other than olfaction.

The presenting the combination of the primary odors in the form recognizable by the perception other than the olfaction may be performed by associating different colors respectively with the primary odors and presenting the odor by a result of mixing the different colors.

The different colors with which the primary odors are respectively associated may be three primary colors.

According to yet another aspect of the present invention, there is provided an apparatus for presenting an odor comprising: a sensor; a gas supply means configured to alternately supply sample gases and reference gas to the sensor, each of the sample gases having an odor corresponding to each of odors in a set of the odors; an information processing device configured to input output signals from the sensor; and an output device configured to output a result of odor presentation obtained by the information processing device by using the above-described method for presenting odor.

Advantageous Effects of Invention

According to the present invention, sample gas that may contain a plurality of chemical substances, that is, an odor (gas generated from the sample when the sample to be given is a liquid or a solid) is analyzed, and samples to be served as primary odors, that is, odors are selected from among the sample gasses. By using the primary odors thus selected, any odor can be represented by the combination of the primary odors. This representation may be one that can be recognized by human perception, such as color (e.g., presentation in a form that can be recognized by perception other than olfaction). Moreover, any odor can also be synthesized at least in an approximate manner.

Brief Description of Drawings

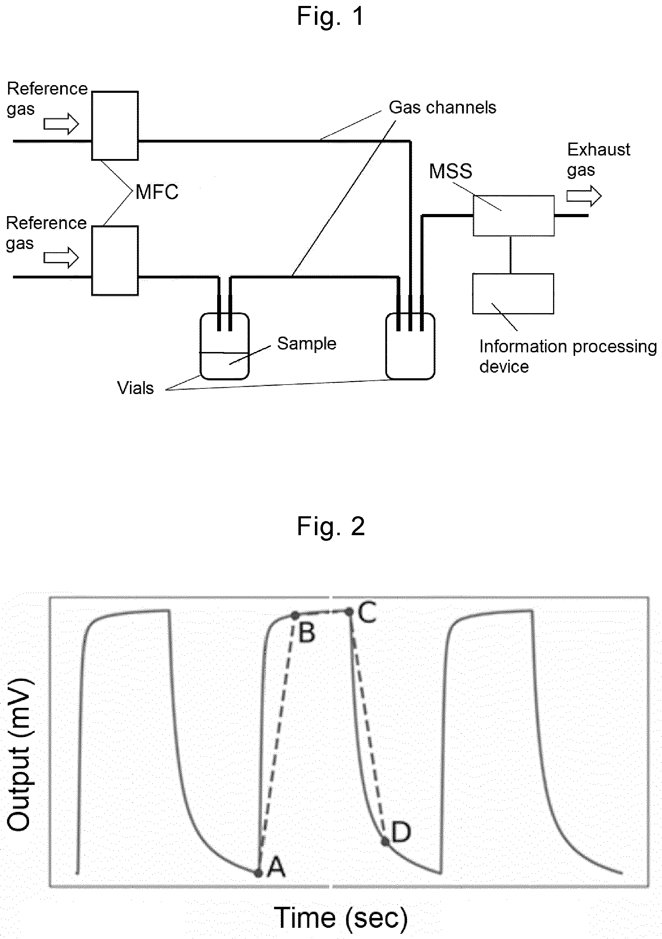

Fig. 1 is a conceptual diagram of a measurement apparatus to which the present invention can be applied.

Fig. 2 is a graph showing an example of response signals measured by a Membrane-type Surface stress Sensor (abbreviated as MSS in the present application). The horizontal axis represents time (unit: second), and the vertical axis represents output (unit: mV). A, B, C, and D are used when the response signals are represented by feature values.

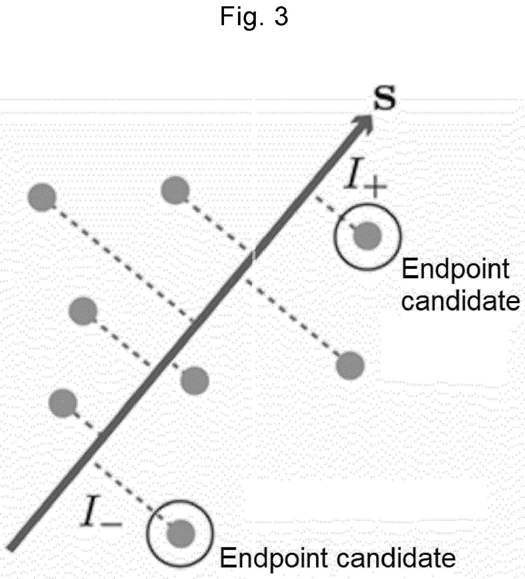

Fig. 3 is an illustration showing an example of an iterative procedure in endpoint detection. In this example, 1 point of an endpoint score is added to each of two points surrounded by a circle.

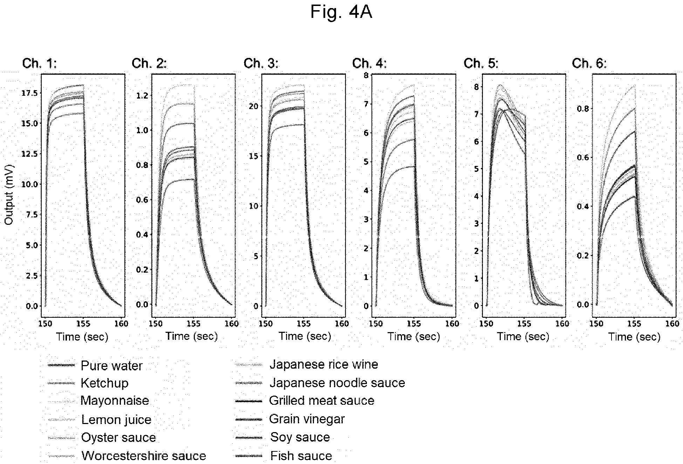

Fig. 4A is graphs showing response signals obtained from channels 1 to 6 among 12 sensing channels of an MSS array. The horizontal axis represents time (unit: second), and the vertical axis represents output (unit: mV). Results obtained by using twelve kinds of seasonings as samples are shown.

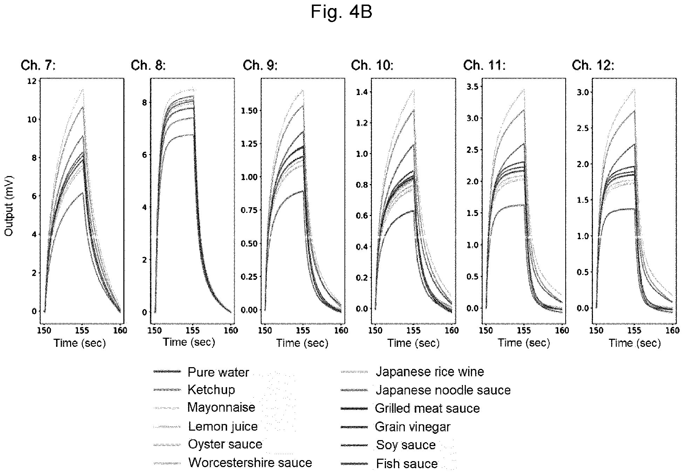

Fig. 4B is graphs showing response signals obtained from channels 7 to 12 among 12 sensing channels of the MSS array. The horizontal axis represents time (unit: second), and the vertical axis represents output (unit: mV). Results obtained by using twelve kinds of seasonings as samples are shown.

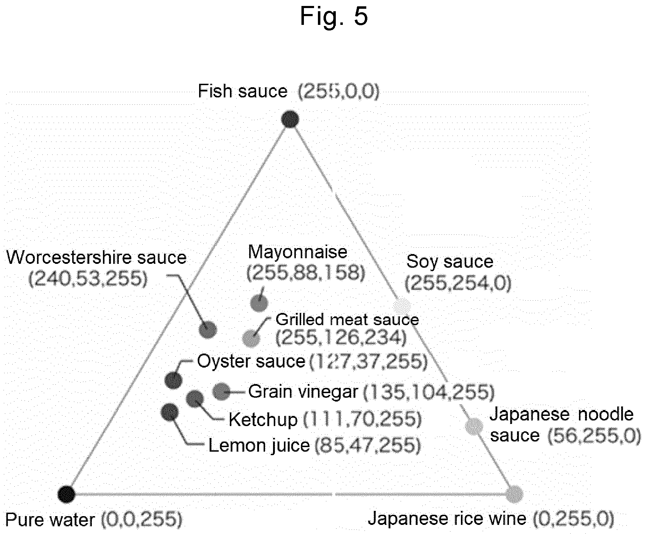

Fig. 5 is an illustration showing a color map for odors of pure water and seasonings obtained in examples. RGB values are shown in parentheses. The result in which fish sauce (nam pla), Japanese rice wine, and pure water were selected as three primary odors by the endpoint detection, and red, green, and blue were respectively assigned as the colors for these primary odors is shown.

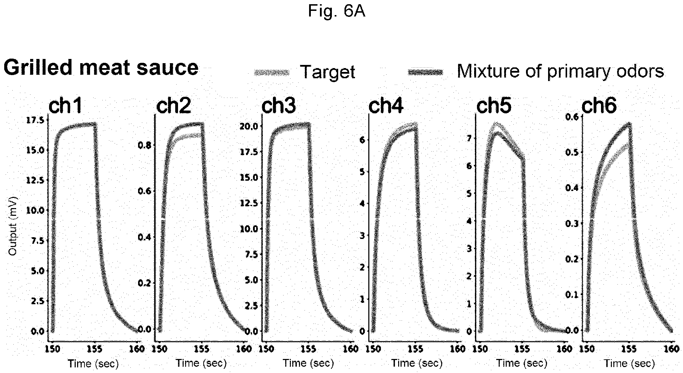

Fig. 6A is graphs for comparing raw response signals and mixed signals with respect to grilled meat sauce in the examples, in which the raw response signals were measured in channels 1 to 6 of the MSS array, and the mixed signals were generated by superimposing response signals of fish sauce, Japanese rice wine, and pure water by using coefficients ($w_1$, $w_2$, $w_3$). The horizontal axis represents time (unit: second), and the vertical axis represents output (unit: mV).

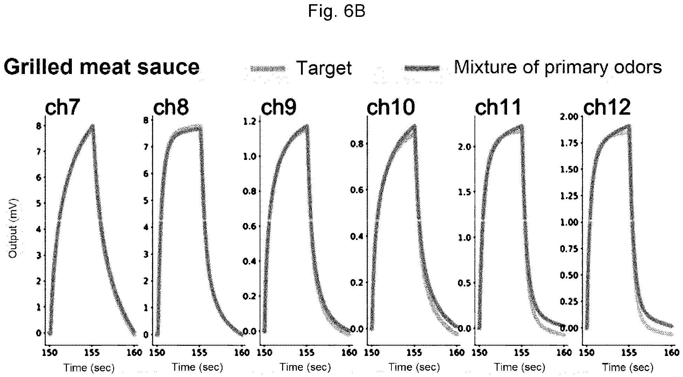

Fig. 6B is graphs for comparing raw response signals and mixed signals with respect to grilled meat sauce in the examples, in which the raw response signals were measured in channels 7 to 12 of the MSS array, and the mixed signals were generated by superimposing response signals of fish sauce, Japanese rice wine, and pure water by using coefficients ($w_1$, $w_2$, $w_3$). The horizontal axis represents time (unit: second), and the vertical axis represents output (unit: mV).

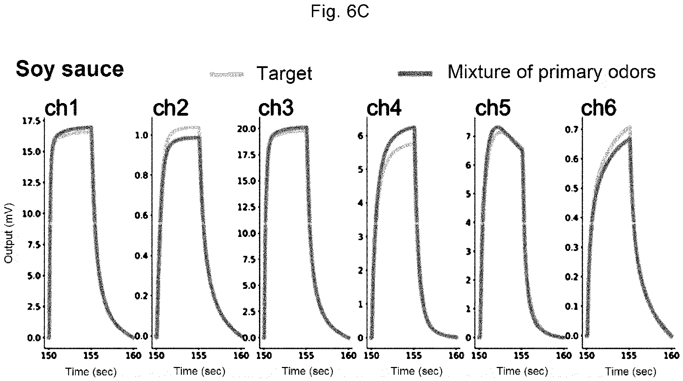

Fig. 6C is graphs for comparing raw response signals and mixed signals with respect to soy sauce in the examples, in which the raw response signals were measured in channels 1 to 6 of the MSS array, and the mixed signals were generated by superimposing response signals of fish sauce, Japanese rice wine, and pure water by using coefficients $(w_1, w_2, w_3)$. The horizontal axis represents time (unit: second), and the vertical axis represents output (unit: mV).

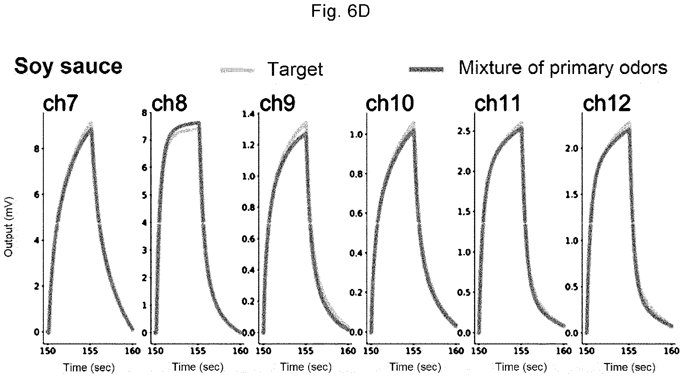

Fig. 6D is graphs for comparing raw response signals and mixed signals with respect to soy sauce in the examples, in which the raw response signals were measured in channels 7 to 12 of the MSS array, and the mixed signals were generated by superimposing response signals of fish sauce, Japanese rice wine, and pure water by using coefficients $(w_1, w_2, w_3)$. The horizontal axis represents time (unit: second), and the vertical axis represents output (unit: mV).

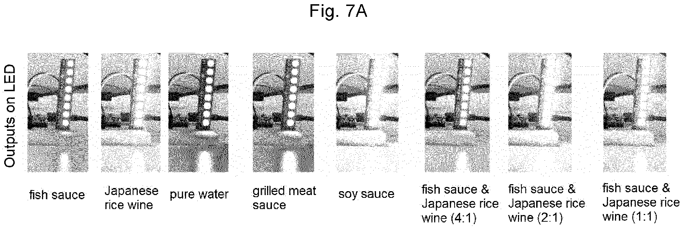

Fig. 7A is photographs showing an example of colors for odors output in real time in the examples.

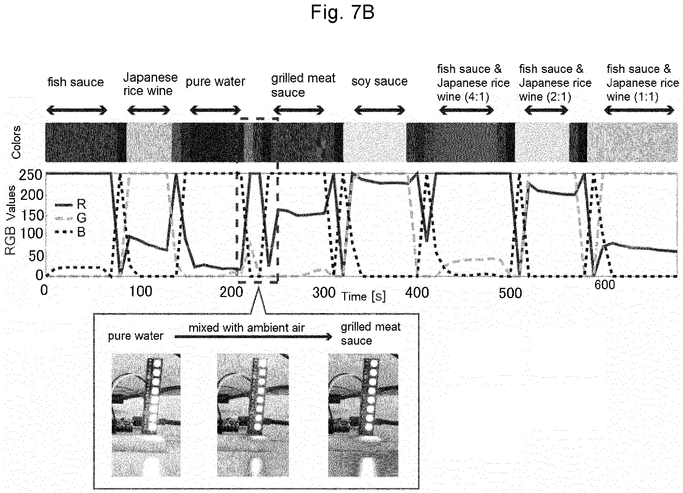

Fig. 7B is an illustration showing an example of colors for odors output in real time and time dependency of RGB values in the examples. The horizontal axis represents time (unit: second), and the vertical axis represents RGB values.

Description of Embodiments

In an embodiment of the present invention, from a set of a plurality of sample gasses (hereinafter, sample gas is also often referred to as "odor"), a subset of these odors is selected by using a nanomechanical sensor and machine learning (When the sample to be given is a liquid or a solid, gas generated therefrom is referred to as sample gas.). The individual odors in the subset of the odors thus selected are each also referred to as a primary odor in the present application. It should be noted that the primary odor is not given a priori (i.e., not given in advance before the application of the present invention) and is dynamically determined in the process of carrying out the present invention, as apparent from the above description. It is a matter of course that once selected primary odors can be reused. For example, in the first measurement, primary odors are selected from a plurality of odors as a subset thereof, and in the subsequent measurement, the primary odors selected in the first time may be used again instead of selecting the primary odors each time. By determining the primary odor in this manner, any odor in a set of given odors can be represented by the combination of odors each serving as a primary odor. The representation of any odor by the primary odors may be provided as mere data (for example, data in which a primary odor 1, a primary odor 2, ... a primary odor n corresponds to $c_1\%$, $c_2\%$, ... and $c_n\%$, respectively), or may be converted such that the odor can be recognized by perception other than olfaction in order to represent the odor more intuitively. As an example for the latter case, by respectively assigning different colors to primary odors, odor can be represented as a color obtained by mixing the colors respectively assigned to these primary odors. Typically, after selecting three primary odors, three primary colors can be assigned to these primary odors. Furthermore, not only the odor is represented as a combination of the primary odors, but also an odor identical or similar to the odor capable of being represented by using the combination of the primary odors can be synthesized by mixing these primary odors.

In other words, the present invention provides a novel and practical technical idea that can be realized even in a current situation where "true primary odor" has not been found. In this technical idea, the whole of almost infinite information of all odors (response of a sensor to all odors) is not targeted, but a computable partial range is cut out from the whole information, and the primary odor is logically defined in this range. Furthermore, even if a true primary odor can be identified, a substance of the primary odor that provides the true primary odor is not necessarily easy to obtain and handle, and is not necessarily safe for a living body. In addition, it is possible that the number of kinds of the primary odors is extremely large. Therefore, it is considered that separation of odor into the primary odors, based on the true primary odors cannot be a realistic solution. The present invention is also sufficiently applicable to such a case.

Describing an embodiment of the present invention more specifically, first, a plurality of sensors that exhibit different responsiveness to various odors are used in order to convert an odor into response signals that can be processed numerically. Examples of such a sensor include a nanomechanical sensor that detects a change in a physical parameter on a predetermined surface thereof. One of the nanomechanical sensors is a surface stress sensor. The surface stress sensor is provided with a film (sensitive film) that adsorbs a specimen such as gas on the surface, and expansion and contraction caused in the sensitive film by adsorption appear as a change in surface stress of the surface. Since various patterns of surface stress changes are measured by using the combination of a substance to be measured and a material of a sensitive film (sensitive film material), qualitative or quantitative analysis of various substances in a sample can be performed by supplying a sample to one or a plurality of surface stress sensors and measuring responses thereof.

Although a variety of sensors for detecting odor have been proposed and produced, a nanomechanical sensor using electrical readings based on piezoresistance, which is referred to below as an MSS (Patent Literature 1 and Non Patent Literature 3), will be used as an example of such a sensor. This sensor has several advantages that have been reported as compared with known cantilever-based nanomechanical sensors and achieves high sensitivity, compactness of its system, and high stability. In addition, the size of the sensor channel is smaller than 1 mm, and a plurality of channels coated with different sensitive film materials can be easily prepared. This makes it possible to acquire a lot of information of the odor at a time, and reliable response signals regarded as a "fingerprint" of the odor sample can be collected. Achievements made by use of the feature value extracted from the response signals (hereinafter, a vector defined by a plurality of feature values is referred to as a feature vector) have been observed in quantitative analysis of odor and odor identification. Of course, other kinds of sensors can also be used.

In order to find a primary odor, first, odor samples are collected and converted into respective response signals by using a plurality of MSS in which various sensitive films are used. Then, a high-dimensional feature vector is extracted from the response signals by applying physicochemical knowledge. Next, by executing endpoint detection using machine learning in high-dimensional feature space, ranking of candidates for primary odors is calculated for all the collected odor samples. The number of odors to be selected as primary odors (the number of primary odors) is determined, and the determined number of primary odors are selected in descending order of the ranking. The other odors are each a mixture of these primary odors. If the target odor can be represented or approximated by a linear combination of feature vectors of these primary odors, coefficients of the linear combination can be calculated by, for example, quadratic programming. In this way, any odor can be represented (approximated) as an odor in which a small number of primary odors are mixed.

General description for the "endpoint detection" mentioned above will be provided. The endpoint detection in the present application means any data analysis method for selecting a sample positioned at an end in the feature space. Various kinds of such machine learning algorithms have been proposed (e.g., Non Patent Literature 4). In the present application, as an example of such a method, a method of finding points located at ends in various directions of the feature space, counting the number of appearances for each point, selecting points in descending order of the number of appearances, and selecting odors associated with these points as primary odors will be described below by applying the method according to Non Patent Literature 5.

In the field of machine learning, the "end" is referred to as an endpoint or an endmember. In accordance with this term, in the present application, a point located at an end of the feature space is defined as an endpoint, and a data analysis method for detecting the endpoint is referred to as endpoint detection. As described above, there are various data analysis methods for detecting an endpoint, and in the present application, a specific method described below is exemplified as one example. However, it should be noted that the endpoint detection is not limited to the specific method, and any data analysis method for detecting an endpoint can be generally used.

In addition to dynamically determining which odor is selected as a primary odor, the number of primary odors may also be dynamically set. For example, in the case where the degree of approximation of an odor other than the primary odors, which is synthesized when a certain number of primary odors are selected, is lower than an expected value, the accuracy of approximation can also be improved by sequentially adding, to the primary odors, higher-order odors that are listed in the above-described ranking and have not been selected, instead of setting the number of primary odors to a preset fixed number. That is, by determining the intensity (weight) of each primary odor and mixing them, an odor other than the primary odors can be synthesized (approximately in many cases). Such synthesis can be implemented by actually mixing the primary odors based on the ratio of the above weight. Alternatively, instead of actually synthesizing an odor, an odor other than the primary odors can be represented on the data as a combination of the selected primary odors. Such representation can be implemented by numerically approximating the feature vector of the odor to be approximated as a linear combination of the primary odors. Examples of such approximation include reconstructing the feature vector of the odor to be approximated as a linear combination of the feature vectors of the primary odors. The degree of approximation between the synthesized or approximated odor and the target to be synthesized or approximated, that is, the target odor, may be determined by a sensory test. Alternatively, a difference between feature vectors of both odors or a difference between response signals themselves of the sensors may be calculated.

The number of primary odors can be set to any number in accordance with the purpose of use of the primary odor. For example, when an odor is displayed in association with a color, the top three samples in the ranking are defined as three primary odors. These primary odors are assigned to three primary colors of red, green, and blue, respectively. However, it is not essential to use three primary colors as colors with which the primary odors are respectively associated, and any three colors can be assigned as the colors for the three primary odors. In this case, the position on the chromaticity coordinate of the target odor is calculated, and the color for the target odor is acquired from the position on the chromaticity coordinate. An apparatus for outputting an odor in a single color in real time can be produced by using, for example, an MSS, a compact computer that implements a machine learning method, an LED light, or the like.

A specific method for selecting primary odors will be described below.

[Feature value extraction from response signals]

Odors are measured by a sensor array which is an aggregate of MSS (hereinafter, each MSS is also referred to as a channel) coated with different sensitive film materials as sensitive films. In a surface stress sensor such as an MSS, a sample to be measured and a reference fluid (Reference gas in the case of odor measurement. When the odor to be measured is supplied to the sensor, the component in the odor is adsorbed to the sensitive film, but the adsorbed component is desorbed while the reference gas flows, and thus reference gas is also called as purge gas in this sense. In the examples, nitrogen gas ($N_2$) is used.) is alternately supplied at a predetermined period, thereby obtaining response signals based on periodic adsorption and desorption occurring in the sensitive film.

A conceptual diagram showing an example of a configuration of a measurement apparatus for performing such measurement is given in Fig. 1. In Fig. 1, the reference gas is supplied to each of the two gas channels from the left side of Fig. 1 (In this paragraph, it is simply referred to as "left side" or "left-side". The same shall apply to the right side, the upper side, and the lower side of Fig. 1). A mass flow controller (MFC) that alternately operates (i.e., gas feeding operation is performed in phases opposite to each other) and feeds gas having a predetermined flow rate toward the downstream side during the operation period is inserted into each channel. With this configuration, during the operation period of the MFC for the upper-side gas channel, the reference gas is directly supplied to the right-side vial in the upper-side gas channel. In the lower-side gas channel, the reference gas from the MFC is supplied to the headspace of the upstream-side vial, and the sample gas evaporated from the sample (liquid sample is assumed here) contained in the vial and accumulated in the headspace is fed toward the downstream via the gas channel. Therefore, during the operation of the MFC for the lower-side channel, the sample gas diluted with the reference gas is fed into the downstream-side vial (the above-described right-side vial). In this way, the reference gas and the sample gas are alternately supplied to the MSS (sensor array including a plurality of MSS) shown in Fig. 1. In response to this, response signals from each MSS in the sensor array are sent to the information processing device and subjected to various kinds of processing to be described later. In order to measure a plurality of samples, it is necessary to replace the samples contained in the vials, if needed. This sample exchange may be performed manually, or the sample may be automatically supplied into and removed from the vial. Alternatively, odor generation containers containing respective samples may be prepared in advance, and these containers may be replaced manually or automatically. A series of odor measurement can also be performed by connecting in advance all the above-described odor generation containers to a large number of provided gas channels, switching the channels by MFC operation, valve control, or the like, and sequentially selecting the odor generation containers. In the information processing device to which the measurement results of the plurality of odors thus obtained are supplied, selection of primary odors, representation of odors based on the selected primary odors, presentation of odors, and the like are performed. The information processing device can be provided with various storage devices and interfaces that can be used when the information of selected primary odors and the odors represented by the primary odors is stored or when such information is supply to other devices. The information processing device can also be provided with an output device such as a display device for presenting the odor to the user in some form. In addition, the odor synthesis by using the primary odors can be performed by, for example, causing the information processing device to appropriately switch the gas channels respectively connected to the plurality of odor generation containers as described above and mix the respective sample gases obtained therefrom. Of course, the configuration shown in Fig. 1 is merely an example, and various modifications can be made, if needed.

More specifically describing such information processing, first, the response signals as shown in Fig. 2 are obtained from each channel coated with a different sensitive film material. In each of the response signals, a peak appears for every predetermined period. These peaks are assumed to be independent of one another. Then, for each peak, the following four parameters representing the shape of the peak are extracted. As a matter of course, the method for extracting the feature value need not be limited thereto.

- Parameter 1: Defined by a gradient between a point A between a point B shown in Fig. 2. The point A corresponds to the time point of switching from the reference gas to the sample gas (odor). The point B is a point where the response signals approach a saturation value due to continuous supply of the odor. This is related to the adsorption process of the odor. As a specific method of defining the point B, a point on the response signals at a time point when a predetermined time has elapsed from the point A may be set as the point B. As an example of another definition, the point B may be set to a point at which, as a result of an increase, the value of the response signals reach a value obtained by: minimum value of the response signals + (maximum value of the response signals - minimum value of the response signals) $\times$ predetermined ratio (e.g., 95%).
- Parameter 2: Defined by a gradient between a point B and a point C corresponding to a time point at which the gas to be supplied is switched from the sample gas (odor) to the reference gas. This includes information about the quasi-equilibrium state.
- Parameter 3: Defined by a gradient between the point C and a point D. The point C is the starting point of the desorption process of the sample gas (odor). The point D is a point at which the value of the response signals approach the minimum value of the response signals due to continuous supply of the reference gas. As a specific method of defining the point D, similarly to defining the point B, a point on the response signals at a time point when a predetermined time has elapsed from the point C may be set as the point D. Alternatively, the point D may be set to a point at which, as a result of a decrease, the value of the response signals reach a value obtained by: minimum value of the response signals + (maximum value of the response signals - minimum value of the response signals) $\times$ predetermined ratio (e.g., 5%).
- Parameter 4: Defined by the height of the peak. This includes information of the adsorption capability of each sensitive film material. In the peak shape shown in Fig. 2, the height of the point C is the parameter 4. However, in general, the magnitude of the output signal does not necessarily increase monotonically during the supply of the sample. In some cases, the magnitude of the output signal reaches the maximum value, that is, the height of peak, at a point between the point B and the point C, and the output signal decreases from this maximum value point toward the future.

The shape of the above peak is not actually constant, and may gradually change from immediately after the start of alternate supply of the sample gas (odor) and the reference gas. Therefore, as for an example of the peak from which the parameter is extracted, it may be preferable to appropriately select a peak that appears after the alternate supply is repeated a sufficient number of times from the start of the alternate supply.

In addition, each sample gas (odor) may be gas from the beginning. Alternatively, the odor generation source may be a liquid or a solid. In the latter case, gas evaporated from the liquid or the solid may be set to a target to be measured. In the embodiment, the odor generation source is described as a liquid, but this description does not lose generality.

[Endpoint detection method for selecting primary odors]

In order to determine a set of primary odors from the collected odor samples, the following endpoint detection method is performed on a high-dimensional feature vector of the odor obtained by measurement using the MSS. Of course, as described above, the endpoint detection method described below is merely an example, and it goes without saying that any other method can be adopted. To simplify the description below, it is assumed that the number of primary odors is three, and the feature values extracted from each odor sample are the parameters 1 to 4 described above with reference to Fig. 2, which are given from each of 12 channels coated with different sensitive films. Therefore, 48 feature values ($12 \times 4 = 48$) are given for each odor sample, and the feature vector has 48 dimensions. As described above, the description is based on the assumption that the number of primary odors and the number of feature values take specific values. However, it is a matter of course that this description does not lose generality.

It is assumed that N is the number of odor samples and

[Mathematical Formula 1]

$$\mathbf{x}_i$$

is a column vector including a 48-dimensional feature of the i-th odor sample. At this time, the feature matrix X is defined as follows.

[Mathematical Formula 2]

$$X = \left(\mathbf{x}_1, \mathbf{x}_2, \cdots, \mathbf{x}_N\right)$$

Each row of the feature matrix X is standardized such that the mean and the standard deviation are 0 and 1, respectively.

Expressed by using a mathematical expression, the above-described standardization is processing in which, for values of each row of the above-described feature matrix $x_{j1}, x_{j2}, ..., x_{ii}, ..., x_{jN}, x_{ji}$ is converted into $(x_{ji} - \mu)/\sigma$. In the above expression, the subscripts i and j and the constants $\mu$ and $\sigma$ are defined as follows.

- i: i is an index of a column of the feature matrix X, and is odor sample numbers 1 to N of the respective odor samples as described above.
- j: j is an index of a row of the feature matrix X, and is associated with the MSS number of the MSS used to measure the value and the parameter number of the parameter obtained from the response signals of the MSS. For example, when 12 MSS (MSS1 to MSS12) are used and four parameters (parameter 1 to parameter 4) are extracted from each MSS as in the above example, j may be defined as: j = (MSS number - 1) $\times$ 4 + parameter number.
- $\mu$: Arithmetic mean of $x_{j1}, x_{j2}, ..., x_{ii}, ..., x_{iN}$.
- $\sigma$: Standard deviation of $x_{j1}, x_{j2}, ..., x_{ii}, ..., x_{jN}$.

This standardization means that each feature vector is translated in the feature space and distributed around the origin of the feature space by setting the mean of each row of the feature matrix to 0. Further, setting the standard deviation of each row to 1 means that the "effectiveness" of individual MSS and parameters, that is, the degree of contribution of individual MSS and parameters to the selection of the primary odor by aligning the MSS to be used and the sizes and variations of the feature values (parameters) obtained from these MSS.

Note that setting the mean of each row to 0 in the above-described standardization does not essentially affect endpoint detection. However, in the machine learning field, standardization in which the mean is set to 0 and the standard deviation is set to 1 (such standardization is also referred to as z-score) is often performed, and thus this standardization is also adopted in this case. Of course, instead of standardization, it is possible to use a normalized value of each row or use the value of each row as it is, and the standardization is not necessarily required. Standardization means to set variance to 1 and mean to 0, and normalization means to convert each row to set the maximum to 1 and the minimum to 0. Additionally, it is not necessarily intended that scale conversion as part of data preprocessing is limited to standardization or normalization, and any scale conversion processing can be applied in an appropriate manner. It should also be noted that such scale conversion is not essential, as a matter of course.

In the endpoint detection method, first, a value K as large as possible (depending on the required accuracy and the like of selection of primary odors, 200 times or more the number of feature values is desirable, for example) is set, and a set of K random unit vectors

[Mathematical Formula 3]

$$\{\mathbf{s}_k\}_{k=1,\ldots,K}$$

is generated. Being "random" means having a randomness that is uniform with respect to directions in feature space (in this case, 48-dimensional vector space). The dimension of each unit vector

[Mathematical Formula 4]

$$\mathbf{s}_k$$

is identical to the dimension of the feature vector, and is 48 dimensions in this case.

Also, a vector

[Mathematical Formula 5]

$$\mathbf{n} = \{n_i = 0\}_{i=1,2,\ldots,N}$$

is prepared. This vector is used to store a score related to the endpoints, called the endpoint score.

In this endpoint detection method, the following procedure is repeated K times.

(1) Feature vectors of all standardized odor data

[Mathematical Formula 6]

$$\mathbf{x}_i$$

are projected onto each unit vector

[Mathematical Formula 7]

$$\mathbf{s}_k$$

. That is, a vector having, as an element, an inner product of the standardized feature vector of each odor

[Mathematical Formula 8]

$$\mathbf{x}_i$$

(wherein, i = 1 to N)
and a given unit vector

[Mathematical Formula 9]

$$\mathbf{s}_k$$

is calculated as follows:

[Mathematical Formula 10]

$$\mathbf{y} = X^{\top}\mathbf{s}_k$$

. This means coordinates in directions of a unit vector

[Mathematical Formula 11]

$$\mathbf{s}_k$$

of the feature vector for each of the odor samples 1 to N. (Note that the feature vector starts at the origin of the feature space.)

(2) Indices $I_-$ and $I_+$ of the minimum and maximum elements among elements (coordinates) in a vector

[Mathematical Formula 12]

$$\mathbf{y}$$

are acquired as follows.

[Mathematical Formula 13]

$$I_- = \arg\min_i \mathbf{y},$$

$$I_+ = \arg\max_i \mathbf{y}.$$

(3) For the indices $I_-$ and $I_+$ of selected element, a component of vector

[Mathematical formula 14]

$$\mathbf{n}$$

is updated as follows. That is, the endpoint score of the element having the selected index is increased by 1.

[Mathematical Formula 15]

$$n_{I_-} \longrightarrow n_{I_-} + 1,$$

$$n_{I_+} \longrightarrow n_{I_+} + 1.$$

An example of this iterative procedure is shown in Fig. 3. When K is sufficiently large, it is considered that the odor sample having the large endpoint score $n_i$ is disposed at an end of high-dimensional feature space (feature vector space). Therefore, a sample with a larger $n_i$ is regarded as a sample positioned at the most end among the odor samples, and the descending order of $n_i$ is considered to be the ranking of candidates for primary odor. In this case, the top three samples are defined as three primary odors, and indices of these primary odors are defined as $e_1$, $e_2$, and $e_3$, respectively. When another odor is represented in color, these primary odors are respectively assigned to the vertices of the chromaticity triangle.

[Method of representing an arbitrary odor using primary odors]

The method for selecting a plurality of primary odors (as an example, three primary odors in the above description; hereinafter, description will be made based on assumption that the number of primary odors is three) among odor samples has been described above in [Endpoint detection method for selecting primary odors]. In order to express another odor by the combination of the primary odors, the feature vector of the other odor is represented by a linear combination of the feature vectors of the three primary odors. At this time, a linear combination in which a feature vector of an odor to be targeted is realized as much as possible is searched. It is assumed that $(w_1, w_2, w_3)$ is coefficients representing a linear combination of a standardized feature vector of three primary odors

[Mathematical formula 16]

$$\mathbf{x}_{e_1}, \mathbf{x}_{e_2}, \text{ and } \mathbf{x}_{e_3}$$

. In the following description, one of the three primary colors of color is associated with corresponding one of the three primary odors, whereby any odor is represented as a mixed color of the three primary colors. However, this description does not also lose generality as a matter of course.

Coefficients representing the linear combination are obtained by executing a quadratic programming. That is, the standardized feature vector of the odor to be targeted is assumed to be

[Mathematical formula 17]

$$\mathbf{x}$$

, coefficients $(w_1, w_2, w_3)$ that minimize a difference $\Delta$ defined by the following formula are obtained.

[Mathematical Formula 18]

$$\Delta = \left\| \mathbf{x} - \sum_{j=1}^{3} w_j \mathbf{x}_{e_j} \right\|^2,$$

Provided, however, that the following two conditions are imposed.

[Mathematical Formula 19]

$$w_j \geq 0$$

$$\sum_{j=1}^{3} w_j = 1$$

The former condition is required to represent the target odor as a positive mixture of three primary odors. This originates from that the primary odor cannot be mixed at a negative concentration. On the other hand, the latter condition is a condition necessary for associating $w_i$ with the concentration at which the primary odor is mixed. When another odor is represented in color, the position of the chromaticity triangle of the target odor is given as follows.

[Mathematical Formula 20]

$$\left(w_1/2 + w_2, \sqrt{3}w_1/2\right)$$

The color associated with the target odor is determined by using the color in the chromaticity triangle corresponding to this position. In this case, the mixed concentration of the primary odor is determined such that the standardized feature vector is approximated, but the target to be approximated is not limited thereto. A normalized feature vector, or a feature vector that is not standardized may be employed. Alternatively, response signals may be employed directly.

Then, the top three samples serving as the candidates for primary odor are regarded as three primary odors, and are used as vertices of a color triangle as in three primary colors of colors, thereby representing all odors with color. As an example of the sample, pure water and seasonings are used in examples. However, it will be obvious from the following description that these samples and targets to be measured are merely examples, and various other kinds of samples and chemical substances can be freely selected. Furthermore, it will be also obvious that the present invention can also be applied to a liquid or solid sample that generates odor.

Examples

[Feature extraction from response signals and used sensitive film materials]

The parameters 1 to 4 that are described above with reference to Fig. 2 were extracted. The repetition period of the supply of the sample (odor) and the reference gas was set to 10 seconds, and the time difference between the point A and the point B was defined as 0.5 seconds. Similarly, the time difference between the points C and D was defined as 0.5 seconds.

Silica/titania composite nanoparticles subjected to various surface functionalization, various polymers, and $SiO_2$-C16TAC (silica/hexadecyltrimethylammonium composite particles) were prepared as 12 kinds of sensitive film materials. These were applied to the surface of the MSS by using several coating techniques. The material applied to each channel and the coating method are stated in [Details of examples - production of MSS chip coated with various materials] to be described later.

As described above, one odor sample is represented by 48 parameters (product of four features and 12 channels). This serves as a high-dimensional feature value for each odor sample, that is, a feature vector.

[Selection of primary odors from odors of pure water and seasonings]

In order to show an example, the primary odors are selected from the odors of pure water and seasonings using the MSS and machine learning. In this case, twelve kinds of seasonings were prepared, namely, pure water, ketchup, mayonnaise, lemon juice, oyster sauce, Worcestershire sauce, Japanese rice wine, Japanese noodle sauce, grilled meat sauce, grain vinegar, soy sauce, and fish sauce were used. Each of these liquid samples was put in a separate vial, and the gas accumulated in the headspace in the vial was measured by MSS. In the present embodiment, the $N_2$ gas identical to the reference gas was used as carrier gas for pushing out the gas (odor) accumulated in the headspace and sending the gas to the MSS. The dependency of the response signals with respect to the sensitive film material is shown in Figs. 4A and 4B. The shape of the signals barely varies in each channel, except for a channel 5 coated with silica/titania composite nanoparticles modified with an aminopropyl group. On the other hand, the height of the peak varies depending on the sample. Japanese rice wine always shows the maximum peak, and fish sauce shows the minimum peak. Therefore, from the viewpoint of raw response signals, Japanese rice wine and fish sauce are considered as candidates for primary odors. On the other hand, for the other odor samples, selection of candidates for primary odors is difficult only by observing the raw response signals. The intensity of the response signals obtained in each channel is different for each channel. In Figs. 4A and 4B, the peak value of the response signals in each channel is set to have a substantially identical height, thereby improving the browsability.

From the response signals obtained from the MSS, 48-dimensional features were extracted. Then endpoint detection was performed, and three primary odors were selected from the prepared odor data. As the endpoint detection method, the example described in [Endpoint detection method for selecting primary odors] was used. The parameter used in the endpoint detection is set to K = 10,000, and the total endpoint score is 20,000. The ranking of the endpoint scores is shown in the table below. The top three samples are fish sauce, Japanese rice wine, and pure water. Half or more of the total endpoint score is occupied by the top two odors, and it is considered that fish sauce and Japanese rice wine are reliably positioned at the endpoints among the prepared pure water and seasonings. This is consistent with the fact confirmed from the raw response signals. On the other hand, the endpoint score of the third-ranked pure water is a relatively small value as compared with these two odors. In such a case, it is difficult to directly find this rank by visual observation, simple comparison between feature vectors, or the like. The present invention is characterized in that it is possible to cope with such a case by performing endpoint detection. Furthermore, since the endpoint scores of grilled meat sauce, oyster sauce, and Worcestershire sauce are considerably small in the data of examples, it can be seen that these odors are located at a deeper area inside the region where the feature vectors are distributed in the 48-dimensional feature space. In this way, by executing the endpoint detection, the relative position in the feature space among the prepared odors can be find out. From the result of the endpoint detection, the primary odors can be determined to be fish sauce, Japanese rice wine, and pure water in the prepared data set of pure water and seasonings.

[Color representation of odor]

In order to convert the target odor into color, the coefficients ($w_1$, $w_2$, $w_3$) are calculated by quadratic programming. (Details are stated in [Method of representing any odor using primary odors] described above.) These coefficients represent the linear combination of the standardized feature vectors

[Mathematical Formula 21]

$$\mathbf{X}_{e_1}, \ \mathbf{X}_{e_2}, \ \text{and} \ \mathbf{X}_{e_3}$$

of the three primary odors extracted from the response signals. As the three primary odors, fish sauce, Japanese rice wine, and pure water are used as selected above. The following table shows the results of determining these coefficients by quadratic programming. The difference $\Delta$ between the linear combination and the standardized feature vector of the target odor is also listed in the table.

[Table 1]

| Sample | EP score | ($\omega_1$, $\omega_2$, $\omega_3$) | $\Delta$ |
|---|---|---|---|
| Fish sauce | 6275 | (1.000, 0.000, 0.000) | 0.000 |
| Japanese rice wine | 5825 | (0.000, 1.000, 0.000) | 0.000 |
| Pure water | 1917 | (0.000, 0.000, 1.000) | 0.000) |
| Japanese noodle sauce | 1494 | (0.181, 0.819, 0.000) | 12.654 |
| Soy sauce | 1196 | (0.501, 0.499, 0.000) | 16.692 |
| Grain vinegar | 1049 | (0.273, 0.210, 0.517) | 1,7.248 |
| Lemon juice | 854 | (0.219, 0.121, 0.660) | 18.699 |
| Ketchup | 588 | (0.254, 0.160, 0.586) | 20.868 |
| Mayonnaise | 568 | (0.509, 0.176, 0.315) | 17.270 |
| Grilled meat sauce | 132 | (0.414, 0.205. 0.380) | 10.388 |
| Oyster sauce | 80 | (0.303, 0.088, 0.609) | 14.293 |
| Worcestershire sauce | 22 | (0.438, 0.097, 0.465) | 6.384 |

The value of $\Delta$ is the accuracy when the odor sample to be targeted is represented by feature vectors of three primary odors. This means that a sample having a small value of $\Delta$, such as Worcestershire sauce or grilled meat sauce, can be represented relatively accurately by mixing the three primary odors. On the other hand, it has been confirmed that the mean value of $\Delta$ is increased when odors other than those of fish sauce, Japanese rice wine, and pure water are used as the primary odors. This fact means that the endpoint detection works effectively for achievement of better color representation. Further, by using these coefficients, the color for each odor sample, the position of each color in the chromaticity triangle, and the RGB value of each odor are summarized in Fig. 5. Note that, red, green, and blue, all of which are three primary colors of color, are respectively assigned to the colors for fish sauce, Japanese rice wine, and pure water in the original color map, but Fig. 5 is a diagram obtained by converting them into a black-and-white drawing.

This color map indicates that the odors of soy sauce and Japanese noodle sauce do not contain a component of pure water and these odors are represented as a mixture of fish sauce and Japanese rice wine. It should be noted that the color itself has no meaning in the above expression. This is because colors unrelated to the original odor sample were assigned to the three primary odors, and thus the other odor samples were converted to colors by relative evaluation using these colors. On the other hand, it is also possible to give meaning to the color itself by applying the color of the liquid sample itself or a color tailored to human senses to the three primary odors using the prepared data set and by creating a chromaticity triangle by mixing such colors.

In Figs. 6A to 6D, coefficients ($w_1$, $w_2$, $w_3$) obtained for soy sauce and grilled meat sauce are used as an example to compare a mixed signal created by superimposing response signals of fish sauce, Japanese rice wine, and pure water with each of response signals obtained from the actual grilled meat sauce and soy sauce. As can be seen from Figs. 6A to 6D that these peaks are well matched in all channels. This indicates that the raw response signals of the target odor can be approximately separated into the response signals of the three primary odors by using the obtained coefficients ($w_1$, $w_2$, $w_3$) so that the given color can be separated into three primary colors. **In** other words, as described above, the coefficients to be used for linear combination of odors were estimated by using only 48-dimensional features, but it is indicated that the obtained coefficients are approximations that can be regarded as the concentrations used when the primary odors are mixed also from the viewpoint of the raw response signals. Therefore, this information is also useful for making a new odor from the three primary odors. The intensity of the response signals obtained in each channel is different for each channel. In Figs. 6A to 6D, as described with reference to Figs. 4A and 4B, the peak value of the response signals in each channel is set to have a substantially identical height, thereby improving the browsability.

[Apparatus for performing color conversion of odor in real time]

When three kinds of primary odors are determined in advance, various kinds of odors can be immediately converted into a form that can be recognized by perception other than olfaction, such as color, only by executing quadratic programming. Therefore, for example, the color for the target odor (color converted from odor as described above) can be output in real time. By utilizing such a technique, a device for displaying the color for odor in real time was produced using the MSS, a compact computer as an information processing device that implements machine learning, and an LED light. The LED light was used as the device for outputting the color, but of course, any output device may be employed. For three kinds of primary odors, fish sauce, Japanese rice wine, and pure water were used as an example. Further, the colors for the primary odors were set to red, green, and blue. Since the $N_2$ gas was used as the reference gas and the carrier gas at the time of measuring the response signals of the primary odors, the $N_2$ gas was used as the reference gas and the carrier gas also at the time of measuring the target odors. However, air can also be used as reference gas and carrier gas.

The real-time measurement results are shown in Figs. 7A and 7B. The first half of the measurement shown in Fig. 7A is results of sequentially measuring fish sauce, Japanese rice wine, pure water, grilled meat sauce, and soy sauce in real time. In addition, Fig. 7B summarizes the time dependency of the color output to the LED light and the RGB value. In the produced device, switching the sample involves operation in which the vial containing the sample whose measurement has been completed is temporarily detached from the gas channel, and the vial containing the sample to be measured next is connected to the gas channel. Thus, surrounding air enters the vial and is mixed with the odor of the sample. Due to this effect, the coefficients greatly fluctuate at the time of switching the sample, and a color different from the color that the odor to be targeted is supposed to exhibit is output. Thereafter, when the response signals reached equilibrium, it was confirmed that a color close to the color that the target odor is supposed to exhibit shown in Fig. 5 was output. (An example thereof is shown on the lower side of Fig. 7B.) In the latter half of this measurement, the odors of three kinds of mixtures each obtained by mixing fish sauce and Japanese rice wine at respective certain concentrations were measured. Mixed samples having concentrations of 4 : 1, 2 : 1, and 1 : 1 were prepared. It was confirmed that the color to be output was a color between the color for fish sauce and the color for Japanese rice wine, that is, a mixed color of red and green.

[Details of examples - production of MSS chip coated with various materials]

A total of 12 channels (hereinafter, also referred to as Ch) are mounted on the MSS chips used in the present examples, and the material applied to each channel and the coating condition are as follows.

Ch1: Aminopropyl group-modified silica/titania composite nanoparticles (spray coating);
Ch2: Octadecyl group-modified silica/titania composite nanoparticles (inkjet coating, 1 g/L, 400 shots);
Ch3: Phenyl group-modified silica/titania composite nanoparticles (inkjet coating, 1 g/L, 200 shots);
Ch4: Polymethyl methacrylate (inkjet coating, 1 g/L, 300 shots);
Ch5: Aminopropyl group-modified silica/titania composite nanoparticles (inkjet coating, 1 g/L, 600 shots);
Ch6: Octadecyl group-modified silica/titania composite nanoparticles (inkjet coating, 1 g/L, 800 shots);
Ch7: Phenyl group-modified silica/titania composite nanoparticles (inkjet coating, 1 g/L, 500 shots);
Ch8: Silica/hexadecyltrimethylammonium composite particles (inkjet coating, 1 g/L, 1000 shots);
Ch9: Tenax TA (mesh: 20/35) (inkjet coating, 1 g/L, 300 shots, stage temperature: 100°C);
Ch10: Tenax TA (mesh: 20/35) (inkjet coating, 1 g/L, 300 shots, stage temperature: 20°C);
Ch11: Tenax TA (mesh: 60/80) (inkjet coating, 1 g/L, 300 shots, stage temperature: 100°C); and
Ch12: Tenax TA (mesh: 60/80) (inkjet coating, 1 g/L, 300 shots, stage temperature: 20°C).

Various silica/titania composite nanoparticles applied to Ch1, Ch2, Ch3, Ch5, Ch6, and Ch7 were synthesized in accordance with methods that are well known to those skilled in the art and described in Non Patent Literature 6 and Non Patent Literature 7. The silica/hexadecyltrimethylammonium composite particles applied to Ch8 were synthesized in accordance with a method that is also well known to those skilled in the art and described in Non Patent Literature 8. The materials applied to Ch4, Ch9, Ch10, Ch11, and Ch12 were all commercially available, and the purchased materials were used as they were without purification or the like.

Various materials were each applied onto the MSS chip as follows. Ch1 was coated by spraying, and the others were coated by using ink jet. These coating methods are well known to those skilled in the art, but if needed, see Non Patent Literature 7 for details of spray coating and Non Patent Literature 9 for details of inkjet coating. It should be noted that, although any of the materials Ch2 to Ch12 was used for coating by preparing a dispersion liquid or a solution having a concentration of 1 g/L, the conditions such as the number of shots and the stage temperature are different for each channel.

Industrial Applicability

The field of application of the present invention is not limited to odor, and the present invention can be used in any fields as long as it is useful in such a field to select original samples, that is, primary odors, from all kinds of gas, liquid, and solid samples. For example, by using a relatively small number of primary odors, any odor can be represented, presented, or synthesized. In addition, an example of application can be conceived as follows. Original samples (primary odors) are selected from various samples such as exhaled breath, sweat, saliva, tears, and any other body fluid, gas or odor emitted from the body. Then, the odor of any sample to be measured, that is, the odor of any individual, can be represented as a combination of the primary odors selected in this manner. Further, this combination can be represented in a form that can be recognized by other perceptions, for example, color. In addition, the present invention is expected to be widely used in various fields where odors or gases are generated, such as production of food, product design, entertainment, storage, distribution, security, and pharmaceutical fields.

Citation List

Patent Literature

Patent Literature 1: WO 2011/148774 A
Patent Literature 2: WO 00/15268 A1

Non Patent Literature

Non Patent Literature 1: https://www.an.shimadzu.co.jp/prt/ff/ff2020-2.htm
Non Patent Literature 2: https://www.an.shimadzu.co.jp/prt/ff/ff2020-1.htm
Non Patent Literature 3: G. Yoshikawa, T. Akiyama, S. Gautsch, P. Vettiger, and H. Rohrer, "Nanomechanical Membrane-type Surface Stress Sensor" Nano Letters 11, 1044-1048 (2011).
Non Patent Literature 4: https://ieeexplore.ieee.org/document/1411995
Non Patent Literature 5: https://ieeexplore.ieee.org/abstract/document/1576691
Non Patent Literature 6: K. Shiba, T. Sugiyama, T. Takei and G. Yoshikawa, Chem. Commun., 2015, 51, 15854-15857.
Non Patent Literature 7: K. Shiba, R. Tamura, G. Imamura and G. Yoshikawa, Sci. Rep., 2017, 7, 3661.
Non Patent Literature 8: K. Kambara, N. Shimura and M. Ogawa, J. Ceram. Soc. Jpn., 2007, 115, 315-318.
Non Patent Literature 9: K. Shiba, R. Tamura, T. Sugiyama, Y. Kameyama, K. Koda, E. Sakon, K. Minami, H. T. Ngo, G. Imamura, K. Tsuda and G. Yoshikawa, ACS Sensors, 2018, 3, 1592-1600.

**Claims**

1.   A method for selecting primary odors from a set of odors;
     **characterized in that** the method comprises:

        detecting each of the odors in the set of the odors using a sensor; and

selecting a subset consisting of the primary odors from among the odors in the set of the odors, based on detection results of each of the odors, each primary odor being one of the odors in the set of the odors,

wherein:

each of the odors in the set of the odors is a sample gas that may contain a plurality of chemical substances, the primary odors represent any odor in the set of the odors by using a combination thereof, the detecting each of the odors includes obtaining a feature vector corresponding to each of the odors, each of the feature vectors comprising a plurality of features obtained from output signals of the sensor for each of the odors, the selecting the subset consisting of the primary odors includes determining the number of odors to be selected as primary odors, and selecting the determined number of primary odors from a set of the feature vectors respectively corresponding to the odors in the set of the odors, based on a subset of the feature vectors selected by endpoint detection using a data analysis method for selecting a feature vector positioned at an end in feature space including the feature vectors, and the subset of the feature vectors is obtained by counting the number of times the respective feature vectors are endpoints, an endpoint being a point located at an extreme end in a respective direction in the feature space, and extracting the feature vectors from the set of the feature vectors in descending order of the counted number of times.

2. The method for selecting primary odors from a set of odors as claimed in claim 1, wherein each row in a feature matrix is subjected to scale conversion, the feature matrix being constituted by taking the feature vectors obtained corresponding to the respective odors as column vectors thereof.

3. The method for selecting primary odors from a set of odors as claimed in claim 1 or 2, wherein the sensor is a surface stress sensor.

4. The method for selecting primary odors from a set of odors as claimed in any one of claims 1 to 3, wherein

the sensor is a sensor array having a plurality of sensor elements, and
the output signals include individual output signals from the respective plurality of sensor elements.

5. The method for selecting primary odors from a set of odors as claimed in any one of claims 1 to 4, wherein the number of odors to be selected as primary odors is three.

6. A method for synthesizing an odor in a set of odors comprising synthesizing, based on the primary odors selected by using the method for selecting primary odors from a set of odors as claimed in any one of claims 1 to 5, a given odor as an odor obtained by mixing the primary odors selected.

7. A method for representing an odor in a set of odors comprising representing, based on the primary odors selected by using the method for selecting primary odors from a set of odors as claimed in any one of claims 1 to 5, a given odor as a combination of the primary odors selected.

8. The method for representing an odor in a set of odors as claimed in claim 7, wherein the combination of the primary odors is represented by a linear combination of feature vectors of the primary odors.

9. A method for presenting an odor in a set of odors comprising presenting the combination of the primary odors as claimed in claim 7 or 8 in a form recognizable by perception other than olfaction.

10. The method for presenting an odor in a set of odors as claimed in claim 9, wherein the presenting the combination of the primary odors in the form recognizable by the perception other than the olfaction is performed by associating different colors respectively with the primary odors and presenting the odor by a result of mixing the different colors.

11. The method for presenting an odor in a set of odors as claimed in claim 10, wherein the different colors with which the primary odors are respectively associated are three primary colors.

12. An apparatus for selecting primary odors from a set of odors, each of the odors in the set of the odors being a sample gas that may contain a plurality of chemical substances, comprising:

a sensor;

a gas supply means configured to alternately supply sample gases and a reference gas to the sensor, each of the sample gases having an odor corresponding to each of the odors in the set of the odors; and

an information processing device configured to input output signals from the sensor,

**characterized in that** the information processing device is configured to select the determined number of odors of the sample gases as primary odors by using the method for selecting primary odors from a set of odors as claimed in any one of claims 1 to 5, each of the determined number of odors selected corresponding to each element of the subset of the feature vectors obtained by the information processing device.

13. The apparatus as claimed in claim 12, further comprising a means configured to mix the sample gases corresponding to the respective primary odors,

wherein the apparatus is configured to perform the method for synthesizing an odor in a set of odors as claimed in claim 6.

14. The apparatus as claimed in claim 12 or 13, further comprising a means configured to store a result of odor representation obtained by the information processing device by using the method for representing an odor in a set of odors as claimed in claim 7 or 8, or configured to provide the result of odor representation to an outside.

15. The apparatus as claimed in any one of claims 12 to 14, further comprising an output device configured to output a result of odor presentation obtained by the information processing device by using the method for presenting an odor in a set of odors as claimed in any one of claims 9 to 11.

**Patentansprüche**

1. Verfahren zum Auswählen von primären Gerüchen aus einer Reihe von Gerüchen;
**dadurch gekennzeichnet, dass** das Verfahren Folgendes umfasst:

Erfassen jedes der Gerüche in der Reihe von Gerüchen unter Verwendung eines Sensors; und
Auswählen einer Teilmenge, die aus den primären Gerüchen besteht, aus den Gerüchen in der Reihe von Gerüchen auf der Grundlage der Erfassungsergebnisse jedes der Gerüche, wobei jeder primäre Geruch einer der Gerüche in der Reihe von Gerüchen ist,

wobei:

jeder der Gerüche in der Reihe von Gerüchen ein Probengas ist, das eine Vielzahl chemischer Substanzen enthalten kann,
die primären Gerüche jeden Geruch in der Reihe von Gerüchen unter Verwendung einer Kombination davon darstellen,
das Erfassen jedes der Gerüche ein Erhalten eines Merkmalsvektors einschließt, der jedem der Gerüche entspricht, wobei jeder der Merkmalsvektoren eine Vielzahl von Merkmalen umfasst, die aus Ausgangssignalen des Sensors für jeden der Gerüche erhalten werden,
das Auswählen der Teilmenge, die aus den primären Gerüchen besteht, ein Bestimmen der Anzahl von Gerüchen, die als primäre Gerüche ausgewählt werden sollen, und ein Auswählen der bestimmten Anzahl von primären Gerüchen aus einer Reihe der Merkmalsvektoren einschließt, die jeweils den Gerüchen in der Reihe der Gerüche entsprechen, basierend auf einer Teilmenge der Merkmalsvektoren, die durch Endpunkterfassung unter Verwendung eines Datenanalyseverfahrens ausgewählt wurden, um einen Merkmalsvektor auszuwählen, der an einem Ende in einem Merkmalsraum positioniert ist, der die Merkmalsvektoren einschließt, und
die Teilmenge der Merkmalsvektoren erhalten wird, indem die Anzahl von Malen gezählt wird, in denen die jeweiligen Merkmalsvektoren Endpunkte sind, wobei ein Endpunkt ein Punkt ist, der sich an einem äußersten Ende in einer jeweiligen Richtung in dem Merkmalsraum befindet, und indem die Merkmalsvektoren aus der Reihe der Merkmalsvektoren in absteigender Reihenfolge der gezählten Anzahl von Malen extrahiert werden.

2. Verfahren zum Auswählen von primären Gerüchen aus einer Reihe von Gerüchen nach Anspruch 1, wobei jede Zeile in einer Merkmalsmatrix einer Skalierungsumwandlung unterzogen wird, wobei die Merkmalsmatrix gebildet wird, indem die entsprechend den jeweiligen Gerüchen erhaltenen Merkmalsvektoren als Spaltenvektoren davon genommen werden.

3. Verfahren zum Auswählen von primären Gerüchen aus einer Reihe von Gerüchen nach Anspruch 1 oder 2, wobei der Sensor ein Oberflächenspannungssensor ist.

4. Verfahren zum Auswählen von primären Gerüchen aus einer Reihe von Gerüchen nach einem der Ansprüche 1 bis 3, wobei

der Sensor eine Sensoranordnung ist, die eine Vielzahl von Sensorelementen aufweist, und
die Ausgangssignale einzelne Ausgangssignale von der jeweiligen Vielzahl von Sensorelementen einschließen.

5. Verfahren zum Auswählen von primären Gerüchen aus einer Reihe von Gerüchen nach einem der Ansprüche 1 bis 4, wobei die Anzahl von als primäre Gerüche auszuwählenden Gerüchen drei beträgt.

6. Verfahren zum Synthetisieren eines Geruchs in einer Reihe von Gerüchen, umfassend ein Synthetisieren auf der Grundlage der primären Gerüche, die unter Verwendung des Verfahrens zum Auswählen von primären Gerüchen aus einer Reihe von Gerüchen nach einem der Ansprüche 1 bis 5 ausgewählt wurden, eines bestimmten Geruchs als ein Geruch, der durch Mischen der ausgewählten primären Gerüche erhalten wird.

7. Verfahren zum Darstellen eines Geruchs in einer Reihe von Gerüchen, umfassend ein Darstellen auf der Grundlage der primären Gerüche, die unter Verwendung des Verfahrens zum Auswählen von primären Gerüchen aus einer Reihe von Gerüchen nach einem der Ansprüche 1 bis 5 ausgewählt wurden, eines bestimmten Geruchs als eine Kombination der ausgewählten primären Gerüche.

8. Verfahren zum Darstellen eines Geruchs in einer Reihe von Gerüchen nach Anspruch 7, wobei die Kombination der primären Gerüche durch eine Linearkombination von Merkmalsvektoren der primären Gerüche dargestellt wird.

9. Verfahren zum Darstellen eines Geruchs in einer Reihe von Gerüchen, umfassend ein Darstellen der Kombination der primären Gerüche nach Anspruch 7 oder 8 in einer Form, die durch eine andere Wahrnehmung als den Geruchssinn erkennbar ist.

10. Verfahren zum Darstellen eines Geruchs in einer Reihe von Gerüchen nach Anspruch 9, wobei das Darstellen der Kombination der primären Gerüche in einer Form, die durch die andere Wahrnehmung als den Geruchssinn erkennbar ist, dadurch erfolgt, dass den primären Gerüchen jeweils unterschiedliche Farben zugeordnet werden und der Geruch durch ein Ergebnis aus einem Mischen der unterschiedlichen Farben dargestellt wird.

11. Verfahren zum Darstellen eines Geruchs in einer Reihe von Gerüchen nach Anspruch 10, wobei die verschiedenen Farben, mit denen die primären Gerüche jeweils assoziiert sind, drei Primärfarben sind.

12. Einrichtung zum Auswählen von primären Gerüchen aus einer Reihe von Gerüchen, wobei jeder der Gerüche in der Reihe der Gerüche ein Probengas ist, das eine Vielzahl chemischer Substanzen enthalten kann, umfassend:

einen Sensor;
ein Gasversorgungsmittel, das so konfiguriert ist, dass es abwechselnd Probengase und ein Referenzgas dem Sensor zuführt, wobei jedes der Probengase einen Geruch aufweist, der jedem der Gerüche in der Reihe der Gerüche entspricht; und
eine Informationsverarbeitungsvorrichtung, die zum Eingeben von Ausgangssignalen vom Sensor konfiguriert ist,
**dadurch gekennzeichnet, dass** die Informationsverarbeitungsvorrichtung so konfiguriert ist, dass sie die bestimmte Anzahl von Gerüchen der Probengase als primäre Gerüche auswählt, indem sie das Verfahren zum Auswählen von primären Gerüchen aus einer Reihe von Gerüchen nach einem der Ansprüche 1 bis 5 verwendet, wobei jede der bestimmten Anzahl von ausgewählten Gerüchen jedem Element der Teilmenge der Merkmalsvektoren entspricht, die von der Informationsverarbeitungsvorrichtung erhalten wurden.

13. Einrichtung nach Anspruch 12, weiter umfassend ein Mittel, das so konfiguriert ist, dass es die Probengase entsprechend den jeweiligen primären Gerüchen mischt,
wobei die Einrichtung so konfiguriert ist, dass sie das Verfahren zum Synthetisieren eines Geruchs in einer Reihe von Gerüchen nach Anspruch 6 durchführt.

14. Einrichtung nach Anspruch 12 oder 13, weiter umfassend ein Mittel, das so konfiguriert ist, dass es ein Ergebnis einer Geruchsdarstellung speichert, das von der Informationsverarbeitungsvorrichtung unter Verwendung des Verfahrens zum Darstellen eines Geruchs in einer Reihe von Gerüchen nach Anspruch 7 oder 8 erhalten wurde, oder so konfiguriert ist, dass das Ergebnis der Geruchsdarstellung nach außen bereitgestellt wird.

**15.** Einrichtung nach einem der Ansprüche 12 bis 14, weiter umfassend eine Ausgabevorrichtung, die so konfiguriert ist, dass sie ein Ergebnis einer Geruchsdarstellung ausgibt, das von der Informationsverarbeitungsvorrichtung unter Verwendung des Verfahrens zum Darstellen eines Geruchs in einer Reihe von Gerüchen nach einem der Ansprüche 9 bis 11 erhalten wurde.

**Revendications**

**1.** Procédé de sélection d'odeurs primaires parmi un ensemble d'odeurs ;
**caractérisé en ce que** le procédé comprend :

la détection de chacune des odeurs de l'ensemble des odeurs au moyen d'un capteur ; et
la sélection d'un sous-ensemble consistant en les odeurs primaires parmi les odeurs de l'ensemble des odeurs, sur la base des résultats de détection de chacune des odeurs, chaque odeur primaire étant l'une des odeurs de l'ensemble des odeurs,

dans lequel :

chacune des odeurs de l'ensemble des odeurs est un échantillon gazeux qui peut contenir une pluralité de substances chimiques,
les odeurs primaires représentent toute odeur de l'ensemble des odeurs en utilisant une combinaison de celles-ci,
la détection de chacune des odeurs inclut l'obtention d'un vecteur de caractéristique correspondant à chacune des odeurs, chacun des vecteurs de caractéristique comprenant une pluralité de caractéristiques obtenues à partir de signaux de sortie du capteur pour chacune des odeurs,
la sélection du sous-ensemble consistant en les odeurs primaires inclut la détermination du nombre d'odeurs à sélectionner comme odeurs primaires, et la sélection du nombre déterminé d'odeurs primaires parmi un ensemble des vecteurs de caractéristique correspondant respectivement aux odeurs de l'ensemble des odeurs, sur la base d'un sous-ensemble des vecteurs de caractéristique sélectionné par détection de point d'extrémité au moyen d'un procédé d'analyse de données pour sélectionner un vecteur de caractéristique positionné à une extrémité dans un espace de caractéristiques incluant les vecteurs de caractéristique, et
le sous-ensemble des vecteurs de caractéristique est obtenu en comptant le nombre de fois où les vecteurs de caractéristique respectifs sont des points d'extrémité, un point d'extrémité étant un point situé à une extrémité extrême dans une direction respective dans l'espace de caractéristiques, et en extrayant les vecteurs de caractéristique de l'ensemble des vecteurs de caractéristique par ordre décroissant du nombre de fois compté.

**2.** Procédé de sélection d'odeurs primaires parmi un ensemble d'odeurs selon la revendication 1, dans lequel chaque rangée d'une matrice de caractéristique est soumise à une conversion d'échelle, la matrice de caractéristique étant constituée en prenant les vecteurs de caractéristique obtenus correspondant aux odeurs respectives comme vecteurs de colonne de celle-ci.

**3.** Procédé de sélection d'odeurs primaires parmi un ensemble d'odeurs selon la revendication 1 ou la revendication 2, dans lequel le capteur est un capteur de contrainte de surface.

**4.** Procédé de sélection d'odeurs primaires parmi un ensemble d'odeurs selon l'une quelconque des revendications 1 à 3, dans lequel

le capteur est un réseau de capteurs présentant une pluralité d'éléments de capteur, et
les signaux de sortie incluent des signaux de sortie individuels de la pluralité respective d'éléments de capteur.

**5.** Procédé de sélection d'odeurs primaires parmi un ensemble d'odeurs selon l'une quelconque des revendications 1 à 4, dans lequel le nombre d'odeurs à sélectionner comme odeurs primaires est de trois.

**6.** Procédé de synthèse d'une odeur dans un ensemble d'odeurs comprenant la synthèse, sur la base des odeurs primaires sélectionnées en utilisant le procédé de sélection d'odeurs primaires parmi un ensemble d'odeurs selon l'une quelconque des revendications 1 à 5, d'une odeur donnée en tant qu'odeur obtenue par le mélange des odeurs primaires sélectionnées.

7. Procédé de représentation d'une odeur dans un ensemble d'odeurs comprenant la représentation, sur la base des odeurs primaires sélectionnées en utilisant le procédé de sélection d'odeurs primaires parmi un ensemble d'odeurs selon l'une quelconque des revendications 1 à 5, d'une odeur donnée en tant que combinaison des odeurs primaires sélectionnées.

8. Procédé de représentation d'une odeur dans un ensemble d'odeurs selon la revendication 7, dans lequel la combinaison des odeurs primaires est représentée par une combinaison linéaire de vecteurs de caractéristique des odeurs primaires.

9. Procédé de présentation d'une odeur dans un ensemble d'odeurs comprenant la présentation de la combinaison des odeurs primaires selon la revendication 7 ou la revendication 8 sous une forme reconnaissable par une perception autre que l'olfaction.

10. Procédé de présentation d'une odeur dans un ensemble d'odeurs selon la revendication 9, dans lequel la présentation de la combinaison des odeurs primaires sous la forme reconnaissable par la perception autre que l'olfaction est effectuée en associant respectivement différentes couleurs aux odeurs primaires et en présentant l'odeur par un résultat de mélange des différentes couleurs.

11. Procédé de présentation d'une odeur dans un ensemble d'odeurs selon la revendication 10, dans lequel les différentes couleurs auxquelles les odeurs primaires sont respectivement associées sont les trois couleurs primaires.

12. Appareil de sélection d'odeurs primaires parmi un ensemble d'odeurs, chacune des odeurs dans l'ensemble des odeurs étant un échantillon gazeux qui peut contenir une pluralité de substances chimiques, comprenant :

   un capteur ;
   un moyen d'alimentation en gaz configuré pour fournir alternativement des échantillons gazeux et un gaz de référence au capteur, chacun des échantillons gazeux présentant une odeur correspondant à chacune des odeurs dans l'ensemble des odeurs ; et
   un dispositif de traitement d'informations configuré pour entrer des signaux de sortie du capteur,
   **caractérisé en ce que** le dispositif de traitement d'informations est configuré pour sélectionner le nombre déterminé d'odeurs des échantillons gazeux en tant qu'odeurs primaires en utilisant le procédé de sélection d'odeurs primaires parmi un ensemble d'odeurs selon l'une quelconque des revendications 1 à 5, chaque odeur du nombre déterminé d'odeurs sélectionnées correspondant à chaque élément du sous-ensemble des vecteurs de caractéristique obtenu par le dispositif de traitement d'informations.

13. Appareil selon la revendication 12, comprenant en outre un moyen configuré pour mélanger les échantillons gazeux correspondant aux odeurs primaires respectives,
   dans lequel l'appareil est configuré pour réaliser le procédé de synthèse d'une odeur dans un ensemble d'odeurs selon la revendication 6.

14. Appareil selon la revendication 12 ou la revendication 13, comprenant en outre un moyen configuré pour stocker un résultat de représentation d'odeur obtenu par le dispositif de traitement d'informations en utilisant le procédé de représentation d'une odeur dans un ensemble d'odeurs selon la revendication 7 ou la revendication 8, ou configuré pour transmettre le résultat de représentation d'odeur vers l'extérieur.

15. Appareil selon l'une quelconque des revendications 12 à 14, comprenant en outre un dispositif de sortie configuré pour délivrer un résultat de présentation d'odeur obtenu par le dispositif de traitement d'informations en utilisant le procédé de présentation d'une odeur dans un ensemble d'odeurs selon l'une quelconque des revendications 9 à 11.

# Fig. 1

# Fig. 2

Fig. 3

Fig. 4A

Ch. 1: | Ch. 2: | Ch. 3: | Ch. 4: | Ch. 5: | Ch. 6:

Pure water
Ketchup
Mayonnaise
Lemon juice
Oyster sauce
Worcestershire sauce

Japanese rice wine
Japanese noodle sauce
Grilled meat sauce
Grain vinegar
Soy sauce
Fish sauce

Fig. 4B

# Fig. 5

Fig. 6A

**Grilled meat sauce**    Target    Mixture of primary odors

Fig. 6B

Fig. 6C

**Soy sauce** ▭ Target ▬ Mixture of primary odors

Fig. 6D

Fig. 7A

Outputs on LED

fish sauce | Japanese rice wine | pure water | grilled meat sauce | soy sauce | fish sauce & Japanese rice wine (4:1) | fish sauce & Japanese rice wine (2:1) | fish sauce & Japanese rice wine (1:1)

## Fig. 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011148774 A
- WO 0015268 A1

**Non-patent literature cited in the description**

- **G. YOSHIKAWA** ; **T. AKIYAMA** ; **S. GAUTSCH** ; **P. VETTIGER** ; **H. ROHRER**. Nanomechanical Membrane-type Surface Stress Sensor. *Nano Letters*, 2011, vol. 11, 1044-1048
- **K. SHIBA** ; **T. SUGIYAMA** ; **T. TAKEI** ; **G. YOSHIKAWA**. *Chem. Commun.*, 2015, vol. 51, 15854-15857
- **K. SHIBA** ; **R. TAMURA** ; **G. IMAMURA** ; **G. YOSHIKAWA**. *Sci. Rep.*, 2017, vol. 7, 3661
- **K. KAMBARA** ; **N. SHIMURA** ; **M. OGAWA**. *J. Ceram. Soc. Jpn.*, 2007, vol. 115, 315-318
- **K. SHIBA** ; **R. TAMURA** ; **T. SUGIYAMA** ; **Y. KAMEYAMA** ; **K. KODA** ; **E. SAKON** ; **K. MINAMI** ; **H. T. NGO** ; **G. IMAMURA** ; **K. TSUDA**. *ACS Sensors*, 2018, vol. 3, 1592-1600